(19) Europäisches Patentamt / European Patent Office / Office européen des brevets

(11) **EP 3 056 234 A1**

(12) **EUROPEAN PATENT APPLICATION**

(43) Date of publication:
17.08.2016 Bulletin 2016/33

(51) Int Cl.:
*A61M 5/145* *(2006.01)*     *A61M 5/31* *(2006.01)*

(21) Application number: 15305196.6

(22) Date of filing: 11.02.2015

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: Fresenius Vial SAS
38590 Brézins (FR)

(72) Inventor: **Couillaud, Frederic**
**38500 Coublevie (FR)**

(74) Representative: **Kusche, Robert et al**
**Fresenius Kabi Deutschland GmbH**
**Patent Department Medical Devices Division**
**Else-Kröner-Straße 1**
**61352 Bad Homburg (DE)**

(54) **Medical device and method for determining a pivoting position of a first part and a second part relative to each other**

(57)     A medical device (1), in particular an infusion device (1), comprises a first part (10), a second part (11) which is pivotable about a pivoting axis (D) with respect to the second part (10), and an evaluation device (14) for evaluating sensor signals. In addition the medical device (1) comprises a first acceleration sensor (13) arranged on the second part (11) and constituted to sense an acceleration (g) acting at the location of the first acceleration sensor (13) and to provide sensor signals indicative of a sensed acceleration (g), wherein the evaluation device (14) is constituted to determine, from the sensor signals provided by the first acceleration sensor (13), a pivoting position of the second part (11) relative to the first part (10). In this way a medical device is provided which allows in an easy and cost-efficient manner to reliably determine the pivoting position of a first part and a second part of the medical device with respect to each other.

## FIG 1B

**(Cont. next page)**

EP 3 056 234 A1

# FIG 4

**Description**

The invention relates to a medical device, in particular an infusion device, according to the preamble of claim 1 and to a method for determining a pivoting position of a first part and a second part of a medical device relative to each other.

A medical device of this kind comprises a first part and a second part pivotable about a pivoting axis with respect to the first part. The medical device furthermore comprises an evaluation device for evaluating sensor signals.

A medical device of this kind may, for example, be constituted as an infusion device, in particular a syringe infusion pump. In this case, the first part may, for example, correspond to a housing of the infusion device, and the second part may correspond to a front door of the infusion device which is pivotable with regard to the housing in order to allow a user access to a syringe arranged on the housing. On the door for example a display may be placed which, during operation of the infusion device, provides operational information about an ongoing infusion process and may also indicate an alarm condition if during the infusion operation a malfunction or the like occurs.

Within a syringe infusion pump, generally the door is closed during an infusion operation. This however is not absolutely necessary, and it may also be possible for a user, for example a nurse, to pivot the door by some degree in order to allow a better visibility of a display on the door. Operation of the syringe pump, however, shall possibly not be allowed in every pivoting position of the door, but only in a defined pivoting range of the door (for example between a fully closed position at 0° and a pivoting angle of for example 40°), because there may be pivoting positions of the door which do not allow a visibility of the display. If the display during operation of the pump is not fully visible to a user, this may be disadvantageous because the user may for example not be able to see an alarm which is displayed on the display.

The infusion device may also be constituted as a volumetric infusion device. In that case on a front face of a housing an infusion set may be placed for interaction with a pump mechanism such that, in a closed state of a door, the infusion set is held in-between the door and the front face of the housing and is in operative connection with the pump mechanism. The door of an infusion device in the shape of a volumetric pump in this regard serves to hold a tubing of an infusion set in a defined position relative to a pump mechanism of the infusion device. A correct infusion operation generally is only possible if the door of the infusion device is closed and the infusion set correspondingly is secured on the infusion device. If the door is opened, this must be detected such that an infusion operation, which potentially may be ongoing, can be stopped and possibly an alarm can be triggered.

With regard to infusion devices, for example with regard to syringe infusion pumps, there hence is a desire to be able to determine a pivoting position of parts of a medical device relative to each other. The determining of a pivoting position of two parts relative to each other herein may be critical, because a correct operation of a medical device may depend on the pivoting position of the parts with respect to each other.

It also may be desired to detect an angular position of a barrel holder within a syringe pump in order to be able to calculate the diameter of a syringe.

The detecting of the pivoting position of two parts of a medical device relative to each other, however, may also be critical in medical devices other than infusion pumps. For example, it may be necessary to monitor a correct functioning of a locking mechanism within a medical device, in particular for those medical devices in which a correct locking of two parts relative to each other is critical for an operation of the medical device.

US 7,311,653 discloses a centrifugal separator having a rotor chamber sealed by a door. Herein, the opening and closing of the door may be sensed by an inclination sensor fixed to the door.

It is an object of the instant invention to provide a medical device and a method which allow in an easy and cost-efficient manner to reliably determine the pivoting position of a first part and a second part with respect to each other.

This object is achieved by means of a medical device comprising the features of claim 1.

Accordingly, the medical device comprises a first acceleration sensor arranged on the second part and constituted to sense an acceleration acting at the location of the first acceleration sensor and to provide sensor signals indicative of a sensed acceleration, wherein the evaluation device is constituted to determine, from the sensor signals provided by the first acceleration sensor, a pivoting position of the second part relative to the first part.

The medical device uses an acceleration sensor to determine the pivoting position of the second part relative to the first part. The acceleration sensor is arranged on the second part and, hence, is pivotable together with the second part relative to the first part. The acceleration sensor senses an acceleration acting at the location at which it is placed on the second part, wherein by pivoting the second part relative to the first part the orientation of the acceleration acting onto the acceleration sensor changes, which can be sensed by the acceleration sensor and can be evaluated for determining the pivoting position of the second part relative to the first part.

Generally, an acceleration due to gravity is present and can be sensed by an acceleration sensor. If, by pivoting the second part relative to the first part of the medical device, the orientation of the acceleration sensor arranged on the second part changes, the direction of gravity sensed by the acceleration sensor will likewise change. From the sensed direction of gravity, it can be concluded at what pivoting position the second part is located, such that the pivoting position of the second part relative to the first part can be determined.

The use of an acceleration sensor on the second part for detecting its pivoting position offers various advantages.

First, the use of an acceleration sensor which senses acceleration and, hence, can sense the direction of gravity allows to determine a pivoting position of a pivotable part in a continuous fashion over a pivoting range. By means of the acceleration sensor it, hence, cannot only be detected whether, for example in the context of an infusion device, a part is opened or closed, but the absolute position of the pivotable part can be determined.

Second, an acceleration sensor generally senses acceleration and, in particular, may sense the direction of gravity. For this, no mechanic or electric contact or no specific spatial relationship of movable parts with respect to each other is required. The acceleration sensor, hence, can be placed inside the pivotable part and can be fully encapsulate within a housing of the pivotable part. This makes the placement of the acceleration sensor easy, ensures for a reliable operation and does not raise any cleaning issues, which is extremely beneficial for medical devices having strict requirements with regard to cleanliness and desinfection.

Third, acceleration sensors such as accelerometers can be obtained as low-cost components and can easily be welded or soldered on a circuit board such as a printed circuit board (PCB).

The evaluation device beneficially is constituted to determine a pivot angel about the pivoting axis in-between the first part and the second part. The evaluation device, thus, determines a relative angle in-between the first part and the second part and, hence, determines by which angle the second part has been pivoted with respect to the first part.

Herein, in one embodiment, the first part is stationary in that during normal use of the medical device it is held fixed and under normal conditions stationary remains at its position. The first part may, for example, be constituted by a housing of an infusion device such as a syringe infusion pump or a volumetric infusion pump.

In contrast to the first part, the second part is pivotable with regard to the first part. The second part may, for example, be a door of a infusion pump which can be pivoted with respect to the first part in order to open the door for placing an infusion set on the infusion device, or to close the door to secure the infusion set on the infusion device and to bring the infusion device into an operative state.

If the first part is stationary and, during normal operation, assumes a defined position, in principle a single acceleration sensor for sensing the acceleration on the second part is sufficient to determine the pivoting position of the second part relative to the stationary first part. The acceleration sensor in this regard is constituted to sense the acceleration along two perpendicular directions and, hence, may sense acceleration in a two-dimensional plane. The two-dimensional plane in which the acceleration sensor may sense the acceleration herein is beneficially orientated transverse to the pivoting axis about which the second part is pivotable with respect to the first part.

The acceleration sensor may hence sense a vector of gravity and from this may conclude the absolute pivoting position of the second part with respect to the direction of gravity. If the position of the stationary first part is fixed, then also the pivoting position of the second part relative to the first part can be concluded.

The acceleration sensor may, for example, comprise a piezoelectric accelerometer. Such accelerometers are cheap and reliable. By means of a piezoelectric accelerometer a dynamic pressure variation, for example caused by a seismic mass element arranged on a piezo-ceramic component, is converted into an electrical signal, which can be evaluated to conclude upon an acceleration acting onto the sensor.

In principle, also different types of acceleration sensors are applicable, such as micro-electromechanical systems (MEMS).

Beneficially herein, acceleration sensors being sensitive along two perpendicular axes (x and y) are used for sensing an acceleration vector within a two-dimensional plane. In principle, however, also three-dimensional acceleration sensors being sensitive along three perpendicular spatial axes (x, y and z) could be used.

The acceleration sensor, for example, can be placed on a circuit board which is placed within the second part. The acceleration sensor can, for example, be soldered to the circuit board. On the circuit board in addition also the evaluation device in the shape of a dedicated electronic circuitry or implemented by a microprocessor or the like can be arranged.

As said, if the first part is stationary and during normal operation and use of a medical device is in a defined position, in principle a single acceleration sensor on the second part is sufficient to reliably determine the relative position of the second part to the first part. If, however, the position of the first part, in particular with regard to its orientation with respect to the direction of gravity, is not entirely defined and may vary with different operating conditions or different use conditions, it may be beneficial if the first part comprises an additional, second acceleration sensor constituted to sense an acceleration acting at the location of the second acceleration sensor. The second acceleration sensor of the first part, hence, provides an additional sensor signal which, in conjunction with the sensor signal of the first acceleration sensor of the second part, can be evaluated by the evaluation device in order to determine the pivoting position of the second part relative to the first part. By means of the acceleration sensor placed in the first part, the orientation of the first part may be determined. Hence, from the first acceleration sensor of the second part, the orientation of the second part is known, whereas from the second acceleration sensor of the first part the orientation of the first part is known, such that by combining this information the relative pivoting position of the second part with respect to the first part can be determined.

Beneficially, both the first acceleration sensor and the second acceleration sensor each are constituted to sense the acceleration along two sensitive axes corresponding to two perpendicular spatial directions. Each acceleration sensor, hence, is sensitive in a two-dimensional plane, wherein the planes of the acceleration sensors both should beneficially be oriented transverse to the pivoting axis about which the second part is pivotable with respect to the first part.

In principle, the acceleration sensors could also be sensitive along three perpendicular spatial directions, which adds to the complexity of the evaluation, but could improve the accuracy of the system.

The second acceleration device may, for example, be placed on a circuit board located within the first part. The second acceleration device may for example be soldered to a printed circuit board (PCB) of the first part.

A medical device may - without limiting the scope of the invention - for example be an infusion device for performing an infusion operation for administering a medical fluid to a patient. The medical device for example may be a syringe infusion pump or a volumetric infusion pump.

If the instant invention is applied to a syringe infusion pump, the first part may for example correspond to a housing of the infusion device, whereas the second part corresponds to a door of the infusion pump which is pivotable with respect to a front face of the housing. On the housing, a syringe may be placed which is accessible by opening the door. On the door a display may be arranged which is visible from the outside and constituted to display operational information of the infusion device during operation. Operation herein may be possible also with a slightly open door such that the door can be pivoted to some degree in order to bring the display in a position in which it is optimally visible for a user, for example a nurse. If the door however is opened by too large a pivoting angle, for example beyond 40° (0° corresponding to the fully closed door), an infusion operation possibly shall be prohibited because the door is opened too wide, possibly leading to the display being not visible at all, which during operation of the infusion device shall be prevented.

The object is also achieved by a method for determining a pivoting position of a first part and a second part of a medical device relative to each other. The second part herein is pivotable with respect to the first part about a pivoting axis. A first acceleration sensor is arranged on the second part and senses an acceleration acting at the location of the first acceleration sensor and provides sensor signals indicative of a sensed acceleration. The sensor signals provided by the first acceleration sensor are evaluated and, from the sensor signals provided by the first acceleration sensor, a pivoting position of the second part relative to the first part is determined.

The advantages and advantageous embodiments described above for the medical device are applicable also to the method such that it shall be referred to the above.

The idea underlying the invention shall subsequently be described in more detail with regard to the embodiments shown in the figures. Herein:

Fig. 1 A        shows a schematic view of a medical device in the shape of an infusion pump, with a door in a closed position;

Fig. 1B         shows a schematic view of the infusion pump of Fig. 1A, with the door in an opened position;

Fig. 2A-2C      show schematic views of a medical device with a pivotable part in different positions;

Fig. 3          shows a schematic view of the device of Fig. 2A to 2C, with a stationary part in a different position; and

Fig. 4      shows a schematic view of a medical device with acceleration sensors being arranged on a stationary part and a pivotable part.

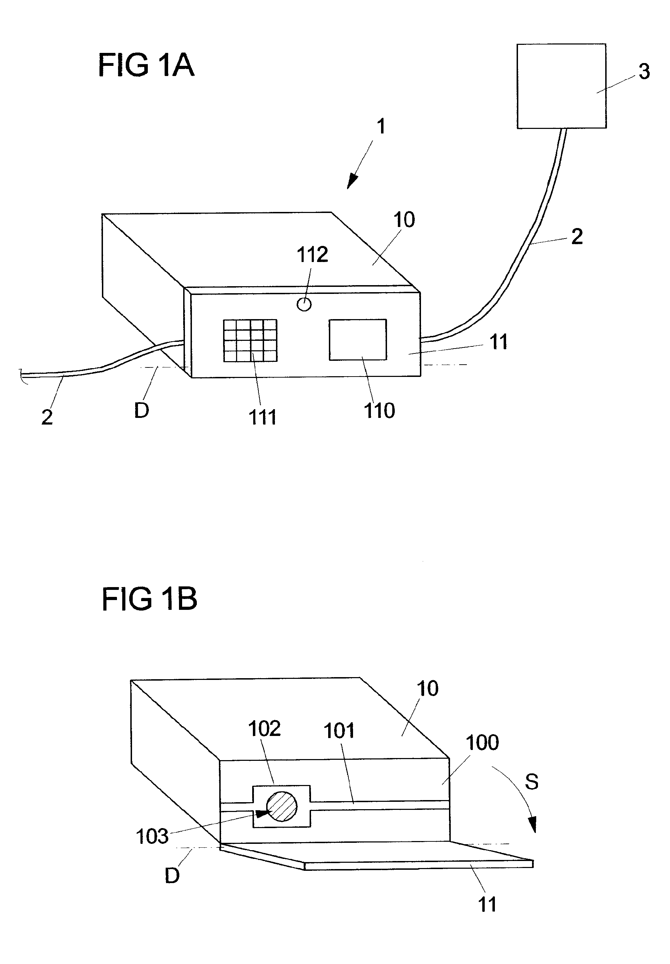

Fig. 1A and 1B show schematic views of a medical device 1 in the shape of an infusion pump. The infusion pump 1 serves to perform a pump action by means of a pump mechanism 103 (see Fig. 1 B) on a tubing of an infusion set 2 in order to pump a medical fluid through the infusion set 2 from a container 3 towards a patient to which the infusion set 2 extends.

In the example of Fig. 1A and 1B the infusion pump 1 is constituted as a volumetric infusion pump. This however is to be understood only as an example. The infusion pump just as well may be a syringe infusion pump or any other infusion device.

The medical device 1 in the shape of the infusion pump comprises a housing 10 which, during normal use of the medical device 1, remains stationary and may, for example, be arranged on a rack for holding the medical device 1 in an organised, mechanically fixed fashion. On the housing 10, a pivotable part called the "door" of the infusion pump is arranged, the pivotable part 11 being pivotable about a pivoting axis D with respect to the housing 10.

The pivotable part 11 can be pivoted about the pivoting axis D in order to pivot the pivotable part 11 along a pivoting direction S between a closed position (Fig. 1A) and an opened position (Fig. 1B). For the case of a volumetric infusion pump, in the closed position a tubing of an infusion set 2 may be held in a secure manner in-between the pivotable part 11 and the housing 10. In the opened position, in contrast, a front face 100 of the housing 10 is accessible such that a tubing of an infusion set 2 can be inserted into a reception grove 101 formed on the front face 100 in order to arrange the infusion set 2 on the medical device 1. On the front face 100 furthermore a reception opening 102 is formed into which, for example, a pump cassette of the infusion set 2 may be inserted for interaction with a pump mechanism 103 arranged within the region of the reception opening 102.

For the case of a volumetric infusion pump, during operation of the medical device 1, the pivotable part 11 is in the closed position as shown in Fig. 1A. In this position, the pump mechanism (see Fig. 1B) acts onto the tubing of the infusion set 2 attached to the medical device 1, for example by acting onto a pump cassette which is integrally formed with the tubing.

Within a syringe infusion pump, the door 11 in its closed state covers a syringe arranged on the housing 10. The door 11 may be opened to access the syringe placed on the housing 10. For the case of a syringe infusion pump, however, the door 11 not necessarily is fully closed during operation of the infusion pump 1, but may be slightly open while the infusion pump 1 is pumping.

As illustrated in Fig. 1A, on the pivotable part 11 of the medical device 1 various components such as a display 110 and an input device, for example in the shape of a keypad comprising a multiplicity of keys, may be arranged.

Furthermore, a latching mechanism 112 is arranged on the pivotable part 11 for locking the pivotable part 11 with respect to the housing 10 in the closed position of Fig. 1A.

As said, during operation of a volumetric infusion pump, the pivotable part 11 must be in the closed position in order to ensure a correct pumping operation of the medical device 1. Hence, if the pivotable part 11 is not in its closed position, a pumping operation of the medical device 1 shall be prevented. If for example during operation of the medical device 1 the pivotable part 11 is unlocked and opened, this must immediately be detected, and possibly an alarm shall be triggered.

For a syringe pump, the door 11 not necessarily must be fully closed during operation. Rather, it may be allowed to pivot the door 11 by some angle for example in order to allow a better visibility of the display 110 to a user. However, the door 11 shall not be opened too wide, because this may actually hinder visibility of the display 110, which must be avoided because alarms may be displayed on the display 110 which must be recognizable by a user.

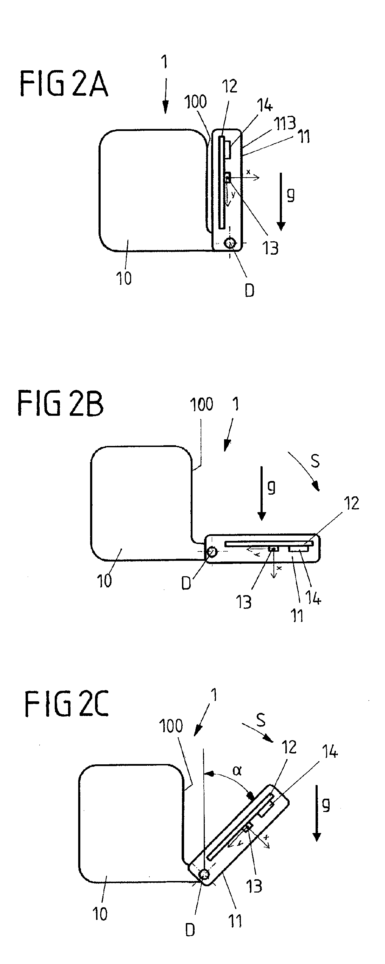

In order to detect whether the pivotable part 11 is in its closed position or at least in an allowed pivoting range (for example between 0° and 40°, 0° corresponding to the fully closed position), an acceleration sensor 13 is arranged on the pivotable part 11, as it is schematically shown in Fig. 2A to 2C. The acceleration sensor 13 is, for example, soldered to a circuit board 12 in the shape of a printed circuit board (PCB) and is encapsulated within a circumferential housing 113 of the pivotable part 11.

The acceleration sensor 13 may, for example, be constituted to be sensitive along two perpendicular spatial axes x, y such that the acceleration sensor 13 can sense an acceleration within a two-dimensional plane spanned by the spatial axes x, y. The acceleration sensor 13 in this way can sense gravity (including its orientation) and the acceleration caused by gravitational forces at the location of the acceleration sensor 13.

Within the embodiment of Fig. 2A to 2C it is assumed that the first part 10 of the medical device 1, for example constituted by a housing of the medical device 1, remains stationary during normal use and operation of the medical device 1 and is at a defined position and orientation which it assumes during operation of the medical device. In this case it is sufficient to place a single acceleration sensor 13 on the second, pivotable part 11 in order to detect a relative pivoting position of the pivotable part 11 with respect to the stationary part 10.

In the closed position as shown in Fig. 2A, the pivotable part 11 is approached to a face 100 of the stationary part 10. Gravitational forces, in this position of the pivotable part 11, act along the vertical axis y of the acceleration sensor 13. From this it can uniquely be concluded that the pivotable part 11 is in its closed position, because only in that position gravitation acts solely along the vertical axis y.

In the opened position of the pivotable part 11, as shown in Fig. 2B, gravitation acts solely along the axis x of the acceleration sensor 13, which in the closed position of the pivotable part 11 (Fig. 2A) had been oriented along a horizontal direction. Due to the pivoting of the pivotable part 11 from the closed position in the pivoting direction S towards the opened positon, the orientation of the axis x however has changed and is now aligned with the direction of gravity, such that in the opened position of the pivotable part 11 an acceleration solely along the x axis of the acceleration sensor 13 is sensed. From this, it is uniquely concluded that the pivotable part 11 is in its opened position.

If the pivotable part 11 assumes a pivoting position in between the fully-closed position and the fully-opened position, the acceleration sensor 13, due to the acting gravity, will sense an acceleration having a component both in the y direction and in the x direction. The acceleration sensor 13 will, hence, output a sensor signal indicating an acceleration which mathematically can be expressed as follows:

$$\overrightarrow{a1} = \underbrace{g.\cos\alpha}_{a}.\vec{y} + \underbrace{g.\sin\alpha}_{b}.\vec{x}$$

Herein, g represents the magnitude of gravity and $\alpha$ represents the angle in between the direction of gravity and the pivotable part 11, and x and y are the basis vectors indicating the sensitive directions of the acceleration sensor 13.

In the above equation, the indicated values a, b are measured and output by the acceleration sensor 13 for the two different spatial directions x, y (value a is output for direction y, whereas value b is output for direction x). Hence, with the measured output of the acceleration sensor 13, $\alpha$ can be determined to be:

$$\alpha = \cos^{-1}\left(\frac{a}{g}\right)$$

which has a unique solution as it can be assumed that $\alpha$ lies in the range between 0° and 180°.

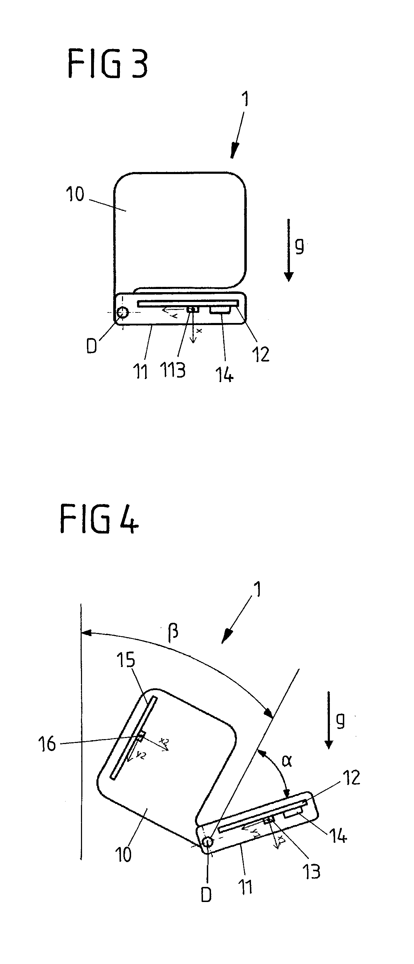

Hence, the relative angle $\alpha$ in between the pivotable part 11 and the stationary part 10 can be determined from the sensor readings of the acceleration sensor 13. This is possible if the stationary part 10 assumes a defined, known position during operation of the medical device 1. If in contrast the stationary part 10 may also assume an arbitrary, non-defined position as illustrated in Fig. 3, by means of a single acceleration sensor 13 located on the pivotable part 11 the relative pivoting position between the pivotable part 11 and the stationary part 10 cannot be accurately determined.

In this case, beneficially a first acceleration sensor 13 is located on the pivotable part 11, and in addition a second acceleration sensor 16 is located on the stationary part 10, as it is illustrated in Fig. 4. The acceleration sensor 16 of the stationary part 10 may, for example, be soldered to a circuit board 15, for example a printed circuit board, placed inside the stationary part 10.

In this case, the two acceleration sensors 13, 16 each sense the acceleration along two perpendicular spatial directions x1, y1 (for the pivotable part 11) and x2, y2 (for the stationary part 10). Using the two acceleration sensors 13, 16, the relative angle $\alpha$ in between the pivotable part 11 and the stationary part 10 can be determined. In particular, the sensor output of the acceleration sensor 16 of the stationary part 10 can be expressed as follows:

$$\overrightarrow{a2} = \underbrace{g.\cos\beta}_{a2}.\overrightarrow{y2} + \underbrace{g.\sin\beta}_{b2}.\overrightarrow{x2}$$

The acceleration reading of the acceleration sensor 13 of the pivotable part 11, in contrast, may be expressed as follows:

$$\overrightarrow{a1} = \underbrace{g.\cos(\alpha+\beta)}_{a1}.\overrightarrow{y1} + \underbrace{g.\sin(\alpha+\beta)}_{b1}.\overrightarrow{x1}$$

Herein, $\alpha$ and $\beta$ are the angels as indicated in Fig. 4; a2, b2 are the sensor readings of the acceleration sensor 16 for the two spatial directions x2, y2, and a1, b1 are the sensor readings for the two spatial directions x1, y1 of the acceleration sensor 13.

From this, the sum of the angles $\alpha$ and $\beta$ can be determined as follows.

$$\alpha + \beta = \cos^{-1}\left(\frac{a1}{g}\right)$$

The angle $\beta$ can be determined as follows:

$$\beta = \cos^{-1}\left(\frac{a2}{g}\right)$$

And from this the relative angle α in-between the pivotable part 11 and the stationary part 10 can be determined to be:

$$\alpha = \cos^{-1}\left(\frac{a1}{g}\right) - \cos^{-1}\left(\frac{a2}{g}\right)$$

which yields the correct solution for α as long α as well as the sum α + β are in the range of 0° and 180°.

Hence, by using two acceleration sensors 13, 16 on the stationary part 10 and the pivotable part 11, the relative angle α in between the pivotable part 11 and stationary part 10 may be determined without knowing the spatial orientation of the stationary part 10 beforehand.

The acceleration sensor 13, 16 may beneficially be placed in an encapsulated fashion within the stationary part 10 and the pivotable part 11 such that they are not accessible from the outside. Hence, no cleaning issue arises from the acceleration sensors 13, 16. Acceleration sensors 13, 16 as described herein may, for example, be constituted as accelerometers, for example MEMS or piezoelectric accelerometers. Such accelerometers can be obtained in a miniaturized form, are cheap and allow for a reliable sensing of an acceleration in space.

The idea underlying the invention is not limited to the embodiments described above, but may be implemented in an entirely different fashion.

In particular, the instant invention is not limited to medical devices in the shape of infusion devices, in particular syringe or volumetric infusion pumps. In principle, the invention may be used on any medical device in which the relative position of a pivotable part with respect to another part shall be sensed.

**List of Reference Numerals**

| | |
|---|---|
| 1 | Medical device (infusion device) |
| 10 | Housing |
| 100 | Front face |
| 101 | Reception groove |
| 102 | Reception opening |
| 103 | Pump mechanism |
| 11 | Pivotable part (door) |
| 110 | Display |
| 111 | Input device |
| 112 | Latch mechanism |
| 113 | Housing |
| 12 | Circuit board |
| 13 | Acceleration sensor |
| 14 | Evaluation device |
| 2 | Infusion set |
| 15 | Circuit board |
| 16 | Acceleration sensor |
| 3 | Container |
| α, β | Angle |
| D | Pivot axis |
| g | Direction of gravity |
| S | Pivot direction |
| x, y; x1, y1; x2, y2 | Basis vectors |

**Claims**

**1.** Medical device (1), in particular infusion device (1), comprising:

- a first part (10),
- a second part (11) which is pivotable about a pivoting axis (D) with respect to the second part (10), and
- an evaluation device (14) for evaluating sensor signals,

**characterized by**

a first acceleration sensor (13) arranged on the second part (11) and constituted to sense an acceleration (g) acting at the location of the first acceleration sensor (13) and to provide sensor signals indicative of a sensed acceleration (g), wherein the evaluation device (14) is constituted to determine, from the sensor signals provided by the first acceleration sensor (13), a pivoting position of the second part (11) relative to the first part (10).

2. Medical device (1) according to claim 1, **characterized in that** the evaluation device (14) is constituted to determine a pivot angle ($\alpha$) about the pivoting axis (D) in between the first part (10) and the second part (11).

3. Medical device (1) according to claim 1 or 2, **characterized in that** the first part (10), in a state of normal use of the medical device (1), is stationary.

4. Medical device (1) according to one of claims 1 to 3, **characterized in that** the second part (11) is pivotable with respect to the first part (10) between a first, closed position, in which the second part (11) is approached towards a face (100) of the first part (10), and a second, opened position, in which the second part (11) is pivoted with respect to the first part (10) such that the face (100) of the first part (10) is accessible by a user.

5. Medical device (1) according to one of the preceding claims, **characterized in that** the first acceleration sensor (13) is constituted to sense the acceleration (g) along two perpendicular directions (x, y).

6. Medical device (1) according to one of the preceding claims, **characterized in that** the first acceleration sensor (13) comprises a piezoelectric accelerometer.

7. Medical device (1) according to one of the preceding claims, **characterized in that** the first acceleration sensor (13) is placed on a circuit board (12) of the second part (11).

8. Medical device (1) according to one of the preceding claims, **characterized in that** the first part (10) comprises an additional, second acceleration sensor (16) constituted to sense an acceleration (g) acting at the location of the second acceleration sensor (16) and to provide sensor signals indicative of a sensed acceleration (g), wherein the evaluation device (14) is constituted to determine, from the sensor signals provided by the first acceleration sensor (13) of the second part (11) and from the sensor signals provided by the second acceleration sensor (16) of the first part (10), a position of the second part (11) relative to the first part (10).

9. Medical device (1) according to claim 8, **characterized in that** the second acceleration sensor (16) is constituted to sense the acceleration (g) along two perpendicular directions (x, y).

10. Medical device (1) according to claim 8 or 9, **characterized in that** the second acceleration sensor (16) comprises a piezoelectric accelerometer.

11. Medical device (1) according to one of claims 8 to 10, **characterized in that** the second acceleration sensor (16) is placed on a circuit board (15) of the first part (11).

12. Medical device (1) according to one of the preceding claims, **characterized in that** the medical device (1) is constituted as an infusion device (1) for performing an infusion operation for administering a medical fluid to a patient, wherein the first part (10) corresponds to a housing (10) of the infusion device (1) and the second part (11) corresponds to a door (11) of the infusion device (1), the door (11) being pivotable about the pivoting axis (D) relative to a face (100) of the housing (10).

13. Method for determining a pivoting position of a first part (10) and a second part (11), which is pivotable with respect to the first part about a pivoting axis (D), of a medical device (1) relative to each other,
   **characterized in**
   **that** a first acceleration sensor (13) arranged on the second part (11) senses an acceleration (g) acting at the location of the first acceleration sensor (13) and provides sensor signals indicative of a sensed acceleration (g), wherein the sensor signals provided by the first acceleration sensor (13) are evaluated and, from the sensor signals provided by the first acceleration sensor (13), a pivoting position of the second part (11) relative to the first part (10) is determined.

**FIG 1A**

**FIG 1B**

FIG 2A

FIG 2B

FIG 2C

FIG 3

FIG 4

Europäisches
Patentamt

European
Patent Office

Office européen
des brevets

# EUROPEAN SEARCH REPORT

Application Number

EP 15 30 5196

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/012802 A1 (FRESENIUS VIAL SAS [FR]) 23 January 2014 (2014-01-23)<br>* page 6, lines 8-16 *<br>* page 7, lines 1-17 *<br>* page 13, lines 1-2 *<br>* figures 1, 2, 10 * | 1-13 | INV.<br>A61M5/145<br><br>ADD.<br>A61M5/31 |
| A | US 2013/146615 A1 (GAUDET MARTIN [CA]) 13 June 2013 (2013-06-13)<br>* paragraphs [0077] - [0078]; figures 1D, 2A * | 5-7 | |
| A | US 2007/100282 A1 (SMALL JAMES R [US] ET AL) 3 May 2007 (2007-05-03)<br>* paragraphs [0057], [0058]; figure 4F * | 1,2,5,13 | |

TECHNICAL FIELDS
SEARCHED (IPC)

A61M

The present search report has been drawn up for all claims

| Place of search | Date of completion of the search | Examiner |
|---|---|---|
| The Hague | 10 August 2015 | Sedy, Radim |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 15 30 5196

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2015

| Patent document cited in search report | | | Publication date | Patent family member(s) | | | Publication date |
|---|---|---|---|---|---|---|---|
| WO 2014012802 | A1 | | 23-01-2014 | CN | 104582754 | A | 29-04-2015 |
| | | | | EP | 2874682 | A1 | 27-05-2015 |
| | | | | US | 2015202362 | A1 | 23-07-2015 |
| | | | | WO | 2014012802 | A1 | 23-01-2014 |
| US 2013146615 | A1 | | 13-06-2013 | NONE | | | |
| US 2007100282 | A1 | | 03-05-2007 | AU | 2006306095 | A1 | 03-05-2007 |
| | | | | CA | 2627379 | A1 | 03-05-2007 |
| | | | | CN | 101309712 | A | 19-11-2008 |
| | | | | EP | 1940490 | A1 | 09-07-2008 |
| | | | | JP | 5676534 | B2 | 25-02-2015 |
| | | | | JP | 2009513266 | A | 02-04-2009 |
| | | | | JP | 2012228574 | A | 22-11-2012 |
| | | | | JP | 2015003262 | A | 08-01-2015 |
| | | | | JP | 2015003263 | A | 08-01-2015 |
| | | | | US | 2007100282 | A1 | 03-05-2007 |
| | | | | WO | 2007050771 | A1 | 03-05-2007 |

EPO FORM P0459

For more details about this annex : see Official Journal of the European Patent Office, No. 12/82

**EP 3 056 234 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 7311653 B